# EUROPEAN PATENT APPLICATION

(11) **EP 1 547 597 A2**
(43) Date of publication of application: **29.06.2005**
(21) Application number: 05001510.6
(22) Date of filing: 10.11.2000
(51) Int. Cl.: A61K 31/506, A61P 25/28

(54) **Use of adatanserin for the treatment of neurodegenerative conditions**

(30) Priority: 12.11.1999 US 439219
(62) Divisional of application: 00979157.5
(71) Applicant: Wyeth, Madison, NJ 07940 (US)
(72) Inventor: Abou-Gharbia, Magid, Abdel-Megid, Princeton Junction NJ 08550 (US); Barrett, James, Edward, Washington Crossing PA 18977 (US); Childers, Wayne, Everett, Jr., New Hope PA 18938 (US); Moyer, John, Allen, New Hope PA 18938 (US)
(74) Representative: Talbott, Dawn Jacqueline

(57) **Abstract**

Adatanserin is useful for treating neurodegenerative disorders, chronic pain, and other disorders associated with dysfunctional glutamate release. Methods of treating the same comprise administering a therapeutically effective amount of adatanserin or a pharmaceutical salt thereof, to a patient in need of said treatment.

## Description

### Background of Invention

Glutamate is the predominant neurotransmitter in the central nervous system and it plays an important role in neuroplasticity. As such, excessive extracellular levels of glutamate have been associated with the pathophysiology of both acute neurodegenerative disorders such as stroke, transient ischemic attack and spinal/brain trauma, as well as chronic neurodegenerative disorders such as epilepsy, Alzheimer's Disease, amyotrophic lateral sclerosis, Huntington's Disease, Parkinson's Disease, AIDS dementia and retinal diseases (Holt, W. F. et al., Glutamate in Health and Disease: The Role of Inhibitors. In: *Neuroprotection in CNS Diseases.* Bar, P. R. and Beal, M. F., ed., Marcel Dekker, Inc., New York **1997**, pp. 87-199; Engelsen, B. A. et al., Alterations in Excitatory Amino Acid Transmitters in Human Neurological Disease and Neuropathology. In: *Neurotoxicity of Excitatory Amino Acids.* Guidotti, A., ed., Raven Press Ltd., New York **1990**, pp. 311 - 332; Ince, P. G. et al., The Role of Excitotoxicity in Neurological Disease. *Res. Contemp. Pharmacother.* **1997**, 8, 195 - 212; Meldrum, B. S. The Glutamate Synapse as a Therapeutical Target: Perspective for the Future. *Prog. Brain. Res.* **1998**, 441 - 458). Compounds which inhibit the release of glutamate would be expected to be useful in the treatment of chronic diseases in which glutamate dysfunction plays a role, such as chronic neurodegeneration, Alzheimer's Disease, Huntington's Disease, Parkinson's Disease, amyotrophic lateral sclerosis, epilepsy, schizophrenia, AIDS dementia and retinal diseases.

Compounds which inhibit or attenuate the release of glutamate would also represent potential neuroprotective agents for the treatment of ischemia resulting from stroke, transient ischemic attack and brain/spinal trauma (Koroshetz, W. J. and Moskowitz, M. A., Emerging Treatment for Stroke in Humans. *Trends in Pharmacol. Sci* **1996**, *17, 227-233;* Dunn, C. D. R. Stroke: Trends, Treatments and Markets. *Scrip Reports,* PJB Publications, Richmond **1995**).

Ischemia can also result from surgery where the blood flow must be halted for a period of time (e.g., cardiac by-pass surgery) due to the resulting anoxia and hypoglycemia (Arrowsmith, J. E. et al., Neuroprotection of the Brain During Cardiopulmonary Bypass. A Randomized Trial of Remacemide During Coronary Artery Bypass in 171 Patients, *Stroke* **1998**, 29, 2357-2362, and references cited within). Compounds which inhibit or attenuate glutamate release would also be expected to show neuroprotective and anti-ischemic activity under these conditions.

Excessive levels of glutamate have been found to be involved in chronic neuropathic or persistent pain, including fibromyalgia, posthepetic neuralgia, reflex sympathetic dystrophy, and diabetic peripheral neuropathy. Meldrum, B.S., *supra.*

Serotonin 5-HT_{1A} receptors are located in brain areas which are highly sensitive to ischemia, such as the hippocampus and cerebral cortex. Activation of this receptor subtype results in neuronal hyperpolarization and a concomitant inhibition of neuronal activity (DeVry, J., 5-HT_{1A} Agonists: Recent Developments and Controversial Issues, *Psychopharmacology,* **1995**, 121, 1-26).

It has been demonstrated that 5-HT_{1A} receptor agonists and partial agonists are able to attenuate glutamate release, most likely through activation of 5-HT_{1A} receptors located on glutamatergic terminals (Matsuyama, S. et al., Regulation of Glutamate Release via NMDA and 5-HT_{1A} Receptors in Guinea Pig Dentate Gyrus. *Brain Res.* **1996**, 728, 175-180). While some 5-HT_{1A} agonists and partial agonists have been shown to exert neuroprotective properties *in vivo* (DeVry, J. et al., BAY x 3702, *Drugs of the Future* **1997**, 22, 341-349, and references cited within) 5-HT_{1A} receptor agonists show varying effects on neuronal survival. (Bode-Greuel, K. M. et al., Serotonin (5-HT_{1A}) Receptor Agonists as Neuroprotective Agents in Cerebral Ischemia. In: *Pharmacology of Cerebral Ischemia 1990,* Krieglstein, J. and Oberpichler, H., ed., Wissenschaftliche Verlagsgesellschaft mgH, Stuttgart (1990), pp. 485-491).

Some serotonin 5-HT₂ antagonists have also been shown to have neuroprotective efficacy. Compounds such as (S)-emopamil (Lin, B. W. et al., (S)-Emopamil Protects against Global Ischemic Brain Injury in Rats. *Stroke* **1990**, 21, 1734-1739; Nakayuama, H. et al., (S)-Emopamil, a Novel Calcium Channel Blocker and Serotonin S2 Antagonist, Markedly reduces Infarct Size Following Middle Cerebral Artery Occlusion in the Rat. *Neurology* **1989**, 38, 1667-1673) and naftidrofuryl (Krieglstein, J. et al., Naftidrofuryl Protects Neurons Against Ischemic Damage. *Eur. Neurology.* **1989,** 29, 224-228; Fujikura, H. et al., A Serotonin S2 Antagonist, Naftidrofuryl, Exhibited a Protective Effect on Ischemic Neuronal Damage in the Gerbil. *Brain Res.* **1989,** 494, 387-390) provide neuroprotective efficacy in animal models of cerebral ischemia.

DE 4138756 teaches 5HT1A receptor agonists, aminomethylchroman derivatives, which enhance the neuroprotective activity of 5-HT2 receptor antagonists such as ketanserin, ritanserin and other 4-fluorophenyl derivatives. (Bode-Greuel, K., Kombination mit Neuroprotektiver Wirkung. German Patent DE 4,138,756, 5/27/93).

Concommitant administration of ipsapirone, a compound having 5-HT1A agonist activity and ketanserin, a compound having 5-HT2 antagonist activity provided more neuroprotection in an animal model of ischemia than either agent alone. (Bode-Greuel, K. M. et al., Serotonin (5-HT_{1A}) Receptor Agonists as Neuroprotective Agents in Cerebral Ischemia. In: *Pharmacology of Cerebral Ischemia 1990,* Krieglstein, J. and Oberpichler, H., ed., Wissenschaftliche Verlagsgesellschaft mgH, Stuttgart (1990), pp. 485-491).

The neuroprotective activity of a compound may be attributed to more than one aspect of its receptor activity profile. For instance, it is hypothesized that for the 5HT1A agonist BAY R 1531, it is not its 5HT1A activity but its low binding affinities for 5HT2, D2 and sigma receptors that may play an important role in its neuroprotective efficacy. Bode-Grueul, *supra.*

Approximately 5-6 million Americans are afflicted with chronic or acute neurodegenerative diseases. Thus, there is a need for an effective compound to treat and prevent neurodegenerative conditions. The present invention provides a useful agent for the treatment and prevention of neurodegenerative disorders.

### Description of Drawings

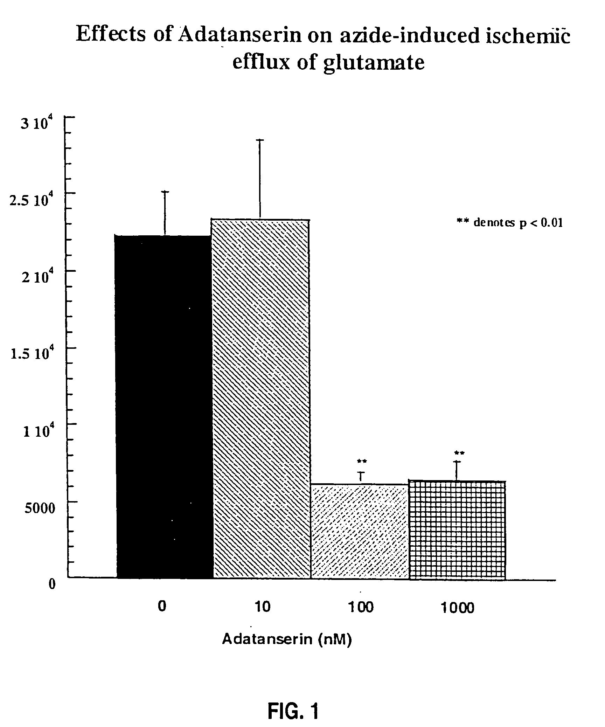

Figure 1 is a schematic representation of the effects of adatanserin on azide-induced ischemic efflux of glutamate in rat hippocampal slices. The effect of three concentrations of adatanserin, 10 (downward diagonal), 100 (upward diagonal) and 1000 nM (checked), were compared to a control (filled). At concentrations of 100 and 1000 nM , a statistically significant reduction of glutamate concentration (72% and 71 % respectively) was observed.

### Detailed Description of the Invention

The present invention relates to novel therapeutic uses of N-[2-[4-(2-pyrimidinyl)-1-piperazinyl]ethyl]tricyclo[3.3.1.13,7]decane-1-carboxamide or adatanserin, and pharmaceutical salts thereof. The present invention provides novel methods of treating chronic and acute neurodegenerative disorders in a mammal in need of such treatment.

Suitable salts can be formed from pharmaceutically acceptable organic and inorganic acids such as hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic and benzenesulfonic acids.

Adatanserin, and pharmaceutical salts thereof, may also be used in the mediation or inhibition of glutamate activity associated with disorders known as chronic, neuropathic or persistent pain.

U.S. Patent No. 5,380,725 and U.S. Patent No. 5,482,940 cover N-[2-[4-(2-pyrimidinyl)-1-piperazinyl]ethyl]tricyclo[3.3.1.13,7]decane-1-carboxamide, or adantanserin, methods of making it and several uses thereof. The uses disclosed are for the treatment of depression, anxiety, psychoses such as paranoia and schizophrenia, the reduction of excess weight and the reduction of excess ethanol consumption. The patents are hereby incorporated by reference.

Dysfunctional glutamate release, and in particular excessive glutamate release, is associated with the pathophysiology of acute and chronic neurodegenerative disorders. Adatanserin has been found to inhibit glutamate release and accordingly is useful for the treatment and prevention of acute and chronic neurodegenerative disorders to ameliorate or eliminate symptoms. A therapeutically effective amount, as used herein, is an amount sufficient to provide a degree of neuroprotection, or to treat, inhibit or ameliorate the symptoms associated with neurodegeneration, chronic pain, or excessive or dysfunctional glutamate release.

Chronic neurodegenerative disorders are, for example, Alzheimer's disease, Huntington's disease, Parkinson's disease, epilepsy, Amyotrophic Lateral Sclerosis, AIDS dementia, and retinal disease.

Acute neurodegenerative disorders include, but are not limited to stroke, head or spinal trauma, and asphyxia.

Stroke includes acute thromboembolic stroke, focal and global ischemia, transient cerebral ischemic attacks and other cerebral vascular problems accompanied by cerebral ischemia.

Other acute neurodegenerative conditions are associated with head trauma, spinal trauma, general anoxia, hypoxia including fetal hypoxia, hypoglycemia, hypotension, as well as similar injuries seen during procedures from embole, hyperfusion, and hypoxia.

The instant invention would also be useful in a range of incidents including during surgery and particularly cardiac surgery, in incidents of cranial hemmorhage, in perinatal asphyxia, in cardiac arrest, and status epilepticus, especially where blood flow to the brain is halted for a period of time.

Chronic, neuropathic or persistent pain includes fibromyalgia, postherpetic neuralgia, reflex sympathetic dystrophy, and diabetic peripheral neuropathy.

Therapeutically effective amounts of adatanserin or pharmaceutical salts thereof, may be administered orally or parentally, neat or in combination with conventional pharmaceutical carriers. Applicable solid carriers can include one or more substances which may also act as flavoring agents, lubricants, solubilizers, suspending agents, fillers, glidants, compression aids, binders, tablet-disintegrating agents or encapsulating materials. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets may contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins. Liquid carriers may be used in preparing solutions, suspensions, emulsions, syrups and elixirs. The active ingredient of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above, e.g., cellulose derivatives, preferably sodium carboxymethyl cellulose solution), alcohols (including monohydric alcohols and polyhydric alcohols, e.g., glycols) and their derivatives, and oils (e.g., fractionated coconut oil and arachis oil). For parenteral administration, the carrier can also be an oily ester such as ethyl oleate and isopropyl myristate. Sterile liquid carriers are used in sterile liquid form compositions for parenteral administration. Liquid pharmaceutical compositions, which are sterile solutions or suspensions can be utilized by, for example, intramuscular, intraperitoneal or subcutaneous injection. Sterile solutions can also be administered intravenously. Oral administration may be either in liquid or solid composition form. Preferably, the pharmaceutical compositions containing the present compounds are in unit dosage form, e.g., as tablets or capsules. In such form, the composition is sub-divided in unit dosages containing appropriate quantities of the active ingredients. The unit dosage forms can be packaged compositions, for example, packaged powders, vials, ampoules, prefilled syringes or sachets containing liquids. Alternatively, the unit dosage form can be, for example, a capsule or tablet itself, or it can be the appropriate number of any such compositions in package form. The therapeutically effective dosage to be used in the treatment of a specific disease or condition must be subjectively determined by the attending physician. Generally, in humans, a daily dosage of from about 100 mg to about 1,500 mg per day may be administered, preferably between about 300 mg and about 1,200 mg per day, more preferably between about 500 mg and 1,000 mg per day. The variables involved in determining an appropriate therapeutic dose include the specific condition(s) being treated and the size, age and response pattern of the patient.

The usefulness of adatanserin and pharmaceutical salts thereof, as agents for the treatment and prevention of neurodegenerative disorders is demonstrated by standard experimental procedures.

### Example 1

### Reduction of ischemic efflux of glutamate in rat hippocampal slices

Adatanserin was evaluated for its ability to reduce the azide-induced ischemic efflux of glutamate in rat hippocampal slices. Three Krebs buffers were used in the experiments. Normal Krebs buffer consisted of a solution containing the following: (122 mM NaCl, 3 mM KCl, 24 mM NaHCO₃, 10 mM glucose, 0.315 mM K₂HPO₄, 1.2 mM MgSO₄, 4 mM CaCl₂). Aglycemic Krebs buffer was similar to the normal buffer with the exception that glucose was not added. Ischemic Krebs buffer was the aglycemic solution which contained varying concentrations of sodium azide (0 - 30 mM).

Rat hippocampi were dissected on a cold platform and suspended in ice cold oxygenated normal Krebs buffer. The tissue was cross-chopped at 350 microns on a Mcllwain tissue chopper, then resuspended and washed three times with ice cold normal Krebs buffer. Approximately 80 mg of tissue (one whole hippocampus) in 130 mL of fluid was added to a well in a Brandel superperfusion apparatus. The samples were perfused with oxygenated normal Krebs buffer and were allowed to equilibrate for 30 minutes at a flow rate of 0.4 mL/minute. Three 10 minute fractions were collected and then ischemia was induced by the application of ischemic Krebs buffer (containing sodium azide). Three additional 10 minute fractions were then collected. For experiments where test compound was examined, drug was added one fraction prior to the induction of ischemia dissolved in aglycemic Krebs buffer. Amino acid concentrations were analyzed with reverse phase HPLC on a catacholamine column (Keystone Scinetific, 150 x 3 mm) employing an 0.05 M acetate/methanol gradient. Alpha-aminoadipic acid was used as an internal standard. Amino acids were visualized by derivatization with naphthalene-2,3-dicarboxaldehyde and fluorimetric detection (Dawson, L. A. et al., Improved Temporal Resolution of Microdialysis Measurement of Glutamate and Aspartate Using Capillary Electrophoresis with Laser Induced Fluorescence Detection. J. Chromatogr. B 1997, 694, 1204-1212). The amino acid concentrations of the first three equilibration fractions were averaged and all subsequent values were expressed as a percentage of this average using the area under the curve (AUC) for each individual experiment as generated by the trapezoid method. Results are presented in Figure 1.

As shown in Figure 1, adatanserin significantly reduced azide-induced ischemic efflux of glutamate in rat hippocampal slices. Concentrations of 100 nM and 1 µM reduced glutamate release by 72% and 71%, respectively.

The present invention may be embodied in other specific forms without departing from the spirit and essential attributes thereof and accordingly, reference should be made to the appended claims, rather than to the foregoing specification, as indicating the scope of the invention.

## Claims

1. A method for treating neurodegenerative disorders comprising administering a therapeutically effective amount of adatanserin or a pharmaceutical salt thereof, to a patient in need of said treatment.

2. The method of Claim 1 wherein the neurodegenerative disorder is chronic.

3. The method of Claim 2 wherein the neurodegenerative disorder is Alzheimer's Disease.

4. The method of Claim 2 wherein the neurodegenerative disorder is Huntington's Disease.

5. The method of Claim 2 wherein the neurodegenerative disorder is Parkinson's Disease.

6. The method of Claim 2 wherein the neurodegenerative disorder is AIDS dementia.

7. The method of Claim 2 wherein the neurodegenerative disorder is Amyotrophic Lateral Sclerosis.

8. The method of Claim 2 wherein the neurodegenerative disorder is retinal disease.

9. The method of Claim 2 wherein the neurodegenerative disorder is epilepsy.

10. The method of Claim 1 wherein the neurodegenerative disorder is acute.

11. The method of Claim 10 wherein the neurodegenerative disorder is stroke.

12. The method of Claim 11 wherein stroke is acute thromboembolic stroke.

13. The method of Claim 11 wherein stroke is focal ischemia.

14. The method of Claim 11 wherein stroke is global ischemia.

15. The method of Claim 11 wherein stroke is transient ischemic attack.

16. The method of Claim 10 wherein the neurodegenerative disorder is ischemia resulting from a surgical technique involving prolonged halt of blood flow to the brain.

17. The method of Claim 10 wherein the neurodegenerative disorder is head trauma.

18. The method of Claim 10 wherein the neurodegenerative disorder is spinal trauma.

19. The method of Claim 10 wherein the neurodegenerative disorder is hypoxia.

20. The method of Claim 19 wherein the hypoxia is fetal hypoxia.

21. A method of treating chronic pain comprising administering a therapeutically effective amount of adatanserin to a patient in need thereof.

22. The method of Claim 21 wherein the pain is diabetic peripheral neuropathy.

23. The method of Claim 21 wherein the pain is selected from fibromyalgia, postherpetic neuralgia, and reflex sympathetic dystrophy.

24. A method of neuroprotection comprising administering a therapeutically effective amount of adatanserin to a patient in need thereof.

25. Use of adatanserin or a pharmaceutical salt thereof, in the preparation of a medicament for the treatment of neurodegenerative disorders.

26. The use of Claim 25 wherein the neurodegenerative disorder is chronic.

27. The use of Claim 25 or Claim 26 wherein the neurodegenerative disorder is Alzheimer's Disease, Huntington's Disease, Parkinson's Disease, AIDS dementia, Amyotrophic Lateral Sclerosis, retinal disease or epilepsy.

28. The use of Claim 25 wherein the neurodegenerative disorder is acute.

29. The use of Claim 28 wherein the neurodegenerative disorder is stroke.

30. The use of Claim 29 wherein stroke is acute thromboembolic stroke, focal ischemia, global ischemia or transient ischemic attack.

31. The use of Claim 28 wherein the neurodegenerative disorder is ischemia resulting from a surgical technique involving prolonged halt of blood flow to the brain.

32. The use of Claim 28 wherein the neurodegenerative disorder is head trauma, spinal trauma or hypoxia.

33. The use of Claim 32 wherein the hypoxia is fetal hypoxia.

34. Use of adatanserin or a pharmaceutical salt thereof, in the preparation of a medicament for the treatment chronic pain.

35. The use of Claim 34 wherein the pain is diabetic peripheral neuropathy.

36. The use of Claim 34 wherein the pain is selected from fibromyalgia, postherpetic neuralgia, and reflex sympathetic dystrophy.

37. Use of adatanserin or a pharmaceutical salt thereof, in the preparation of a medicament for the provision of neuroprotection.
